# EUROPEAN PATENT APPLICATION

(11) **EP 4 425 244 A1**
(43) Date of publication of application: **04.09.2024**
(21) Application number: 22886923.6
(22) Date of filing: 24.10.2022
(51) Int. Cl.: G02C 13/00, C08L 33/04, C08L 55/00, G02C 7/04

(54) **TREATMENT FLUID FOR SOFT CONTACT LENS**

(30) Priority: 29.10.2021 JP 2021177580
(71) Applicant: NOF Corporation, Shibuya-ku Tokyo 150-6019 (JP)
(72) Inventor: GOTANDA Ryuya, Kawasaki-shi, Kanagawa 210-0865 (JP); IWAKIRI Norio, Kawasaki-shi, Kanagawa 210-0865 (JP)
(74) Representative: Mewburn Ellis LLP
(86) International application number: PCT/JP2022/039425
(87) International publication number: WO 2023/074596

(57) **Abstract**

Provided is a treating solution for soft contact lenses capable of simply imparting hydrophilicity and lubricity to a soft contact lens surface, and excellent durability of such hydrophilicity and lubricity thereto. The treating solution for soft contact lenses of the present disclosure is achieved by dissolving a copolymer obtained by copolymerizing at least two specific kinds of monomers in a solvent at a specific concentration.

## Description

### Technical Field

The present disclosure relates to a treating solution for soft contact lenses that can impart excellently sustained hydrophilicity and lubricity to a surface of a soft contact lens by simple treatment, and can provide a soft contact lens with sustained satisfactory wearing feeling lasting all day.

The present application claims priority from Japanese Patent Application No. 2021-177580, which is incorporated herein by reference.

### Background Art

Hydrophilicity and lubricity of a surface of a soft contact lens are deeply involved in wearing feeling of the soft contact lens, and hence, in order to obtain a soft contact lens with sustained wearing feeling lasting all day, it is required that high hydrophilicity and lubricity of the surface of the lens be sustained for a long time period.

In Patent Literature 1, there is a disclosure of a technology involving subjecting a soft contact lens to plasma treatment. In Patent Literature 2, there is a disclosure of a technology involving chemically bonding a soft contact lens having a reactive group and a hydrophilic polymer having a reactive group to each other to hydrophilize a surface of the soft contact lens. Each of the technologies of Patent Literature 1 and Patent Literature 2, which involves treating a soft contact lens itself to impart excellent hydrophilicity to a surface of the soft contact lens, provides excellent hydrophilicity and excellent durability thereof to the soft contact lens, but has room for improvement in lubricity of the soft contact lens. In addition, each of those technologies includes complicated surface treatment steps, and hence highly controlled production equipment is required, leading to an economic disadvantage in some cases.

In each of Patent Literatures 3 and 4, there is a disclosure of a technology involving blending polyethylene glycol, a cellulose-based polymer, or the like into a treating solution for soft contact lenses to impart hydrophilicity to a soft contact lens surface and enhance hydrophilicity thereof, to thereby improve the wearing feeling.

A phosphorylcholine-like group-containing polymer has a phospholipid-like structure derived from a biomembrane, and is known for having excellent characteristics, such as extremely high hydrophilicity and high moisture retention. The technology of Patent Literature 5 is a technology in which the wearing feeling is improved by adding a copolymer of a phosphorylcholine group-containing monomer and butyl methacrylate to a treating solution for soft contact lenses.

### Citation List

### Patent Literature

[PTL 1] WO 2010/092686 A1
[PTL 2] WO 0001/074932 A1
[PTL 3] WO 2009/032122 A1
[PTL 4] WO 2012/098653 A1
[PTL 5] US 2009/0100801 A1

### Summary of Invention

### Technical Problem

A contact lens using the treating solution for soft contact lenses described in each of Patent Literatures 3, 4, and 5 is excellent in wearing feeling, but adsorption of a copolymer to a contact lens surface is not sufficient, and hence improvement in the wearing feeling is temporary.

An object of the present disclosure is to provide a treating solution for soft contact lenses capable of simply imparting hydrophilicity and lubricity to a soft contact lens surface, and excellent durability of such hydrophilicity and lubricity thereto.

### Solution to Problem

The inventors of the present invention have made extensive investigations, and as a result, have found that the above-mentioned object can be achieved by dissolving a copolymer obtained by copolymerizing at least two specific kinds of monomers in a solvent at a specific concentration to provide an excellent treating solution for soft contact lenses. Thus, the inventors have completed the treating solution for soft contact lenses of the present disclosure.

That is, a treating solution for soft contact lenses according to one embodiment of the present disclosure, a surface treatment method for a soft contact lens according to one embodiment of the present disclosure, and a use of a copolymer (P) for production of a treating solution for soft contact lenses according to one embodiment of the present disclosure are as described below.
1. A treating solution for soft contact lenses, including 0.001 w/v% to 2.0 w/v% of a copolymer (P), wherein the copolymer (P) has structural units represented by the following formulae (1a) and (1b), has a molar ratio nₐ:n_{b} between the structural units of 10 to 90:10 to 90, and has a weight-average molecular weight of from 10,000 to 5,000,000: 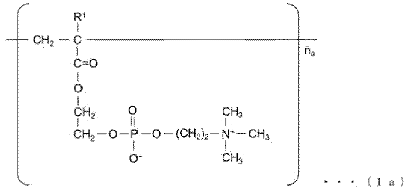 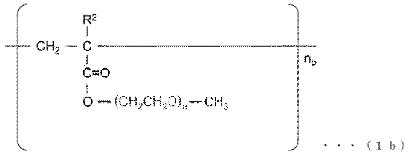 in the formula (1a) and the formula (1b), R¹ and R² each independently represent a hydrogen atom or a methyl group, and in the formula (1b), "n" represents from 4 to 90.
2. The treating solution for soft contact lenses according to the above-mentioned item 1, wherein the structural unit (1a) is 2-(methacryloyloxy)ethyl 2-(trimethylammonio)ethylphosphate.
3. The treating solution for soft contact lenses according to the above-mentioned item 1 or 2, wherein the copolymer (P) is a copolymer formed of the structural units represented by the formulae (1a) and (1b).
4. A surface treatment method for a soft contact lens, the method including a step of bringing a solution for soft contact lenses into contact with a soft contact lens surface, wherein the solution for soft contact lenses contains 0.001 w/v% to 2.0 w/v% of a copolymer (P), the copolymer (P) having structural units represented by the following formulae (1a) and (1b), having a molar ratio na:nb between the structural units of 10 to 90:10 to 90, and having a weight-average molecular weight of from 10,000 to 5,000,000: 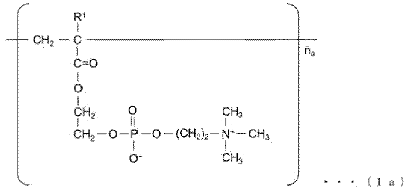 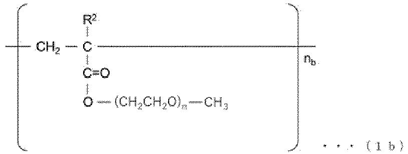 in the formula (1a) and the formula (1b), R¹ and R² each independently represent a hydrogen atom or a methyl group, and in the formula (1b), "n" represents from 4 to 90.
5. A use of a copolymer (P) for production of a treating solution for soft contact lenses, the copolymer (P) having structural units represented by the following formulae (1a) and (1b), having a molar ratio na:nb between the structural units of 10 to 90:10 to 90, and having a weight-average molecular weight of from 10,000 to 5,000,000: 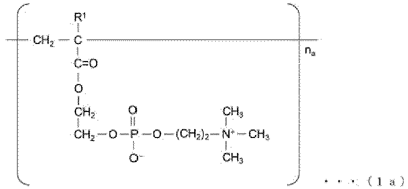 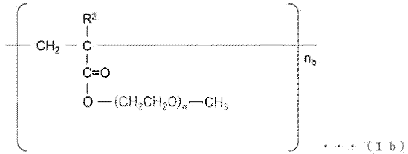 in the formula (1a) and the formula (1b), R1 and R2 each independently represent a hydrogen atom or a methyl group, and in the formula (1b), "n" represents from 4 to 90.

### Advantageous Effects of Invention

The treating solution for soft contact lenses capable of imparting excellent hydrophilicity and lubricity to a soft contact lens, and durability of such hydrophilicity and lubricity thereto can be provided.

### Description of Embodiments

A treating solution for soft contact lenses of the present disclosure includes 0.001 w/v% to 2.0 w/v% of a copolymer (P). The copolymer (P) has structural units represented by the following formulae (1a) and (1b), has a molar ratio nₐ:n_{b} between the structural units of 10 to 90:10 to 90, and has a weight-average molecular weight of from 10,000 to 5,000,000.

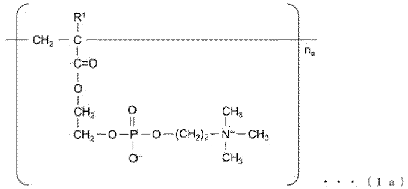

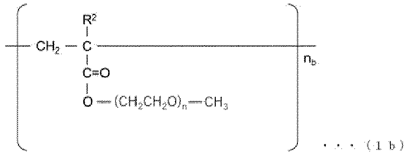

In the formula (1a) and the formula (1b), R¹ and R² each independently represent a hydrogen atom or a methyl group, and in the formula (1b), "n" represents from 4 to 90.

As used herein, the term "(meth)acrylate" means "acrylate or methacrylate", and the same applies to other similar terms.

In addition, herein, when preferred numerical ranges (e.g., the ranges of a concentration or a weight-average molecular weight) are described in stages, the respective lower limit values and upper limit values may be independently combined with each other. For example, in the description: "preferably 10 or more, more preferably 20 or more, and preferably 100 or less, more preferably 90 or less", the "preferred lower limit value: 10" and the "more preferred upper limit value: 90" may be combined to obtain a range of "10 or more and 90 or less". In addition, for example, also in the description: "preferably from 10 to 100, more preferably from 20 to 90", a range of "from 10 to 90" may be similarly obtained.

Herein, the "treating solution for soft contact lenses" (in particular, a shipping solution for soft contact lenses) refers to a solution to be included in a packaging container, such as a blister package, together with a soft contact lens in the distribution of soft contact lenses. In general, a soft contact lens is used in a state of being swollen with an aqueous solution, and hence the lens is packed into a packaging container in a state of being swollen with an aqueous solution at the time of shipping from a factory so as to be readily usable.

### <Copolymer (P)>

The copolymer (P) to be used (contained) in the treating solution for soft contact lenses of the present disclosure has the structural units represented by the following formulae (1a) and (1b), has a molar ratio nₐ:n_{b} between the structural units of 10 to 90:10 to 90 (or a molar ratio nₐ:n_{b} of 100:10 to 900), and a weight-average molecular weight of from 10,000 to 5,000,000.

The "structural unit" means a unit of a compound contained in a polymer based on each monomer or derived from each monomer.

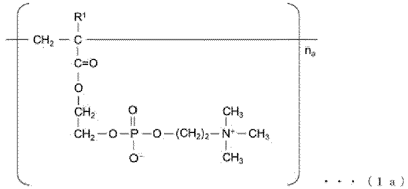

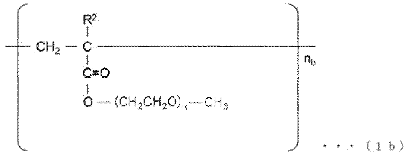

In the formula (1a) and the formula (1b), R¹ and R² each independently represent a hydrogen atom or a methyl group, and in the formula (1b), "n" represents from 4 to 90.

### [Monomer represented by Formula (1a)]

The copolymer (P) to be used in the present disclosure has a structural unit represented by the following general formula (1a). The structural unit is obtained by polymerizing a hydrophilic monomer represented by the following general formula (1a'), that is, a monomer having a phosphorylcholine structure (hereinafter sometimes referred to as "hydrophilic monomer" or "PC monomer"). By virtue of the copolymer (P) containing the PC monomer as a structural unit, hydrophilicity can be imparted to a soft contact lens.

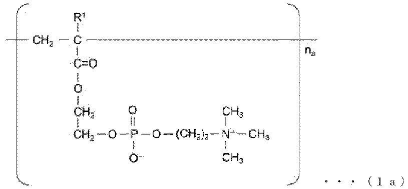

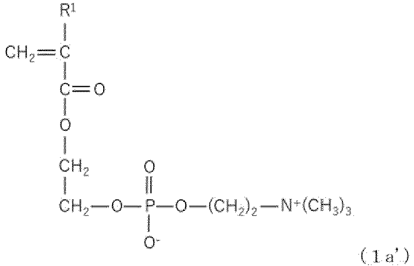

In the formula (1a) and the formula (1a'), R¹ represents a hydrogen atom or a methyl group.

The PC monomer is 2-((meth)acryloyloxy)ethyl 2-(trimethylammonio)ethylphosphate, preferably 2-(methacryloyloxy)ethyl 2-(trimethylammonio)ethylphosphate represented by the following general formula (1a") (hereinafter sometimes referred to as "2-methacryloyloxyethyl phosphorylcholine").

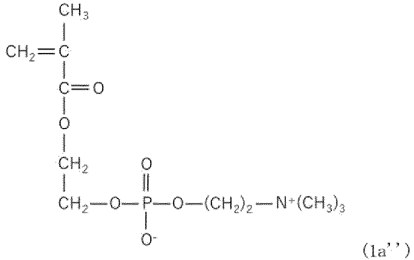

The content of the PC monomer in the copolymer (P) is from 10 mol% to 90 mol%, preferably from 20 mol% to 80 mol%, more preferably from 30 mol% to 70 mol%, still more preferably from 40 mol% to 60 mol% or from 30 mol% to 50 mol%. When the content is less than 10 mol%, a hydrophilicity-improving effect on a soft contact lens surface cannot be expected, and when the content is more than 90 mol%, the amount of the monomer represented by the formula (1b) becomes relatively small, and hence the ability of the copolymer (P) to be adsorbed to the soft contact lens surface is reduced. Accordingly, durability of wearing feeling becomes insufficient.

### [Monomer represented by Formula (1b)]

The copolymer (P) to be used in the present disclosure has a structural unit represented by the following formula (1b). The structural unit includes a monomer represented by the following general formula (1b'), that is, a monomer having a polyethylene glycol structure (hereinafter sometimes referred to as "PME monomer"). By virtue of the copolymer (P) containing the PME monomer, the soft contact lens to be obtained gives sustained satisfactory wearing feeling.

In addition, the copolymer (P) may contain a plurality of the structural units represented by following the formula (1b) having different values of "n".

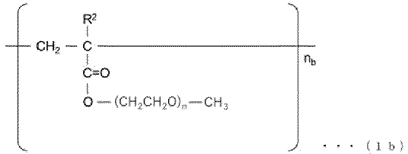

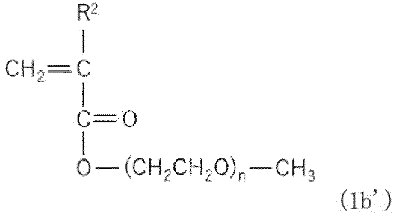

In the formulae (1b) and (1b'), R² represents a hydrogen atom or a methyl group, and "n" represents from 4 to 90.

The content of the PME monomer in the copolymer (P) is from 10 mol% to 90 mol%, preferably from 20 mol% to 80 mol%, more preferably from 30 mol% to 70 mol%, still more preferably from 40 mol% to 60 mol% or from 50 mol% to 70 mol%. As described above, the copolymer (P) to be used in the present disclosure contains the monomer represented by the formula (1b) as a structural unit, and hence the ability of the copolymer (P) to be adsorbed to the soft contact lens surface can be improved. Accordingly, durability of the wearing feeling becomes satisfactory.

In the formula (1b), "n" represents from 4 to 90, preferably from 9 to 60, more preferably from 9 to 30, still more preferably from 20 to 30. When "n" represents less than 4, the ability of the copolymer (P) to be adsorbed to the soft contact lens surface is reduced. When "n" represents more than 90, an aggregation force between molecules of the copolymer (P) increases, and the transparency of the treating solution for soft contact lenses or contact lens to be obtained deteriorates.

The copolymer (P) can achieve both of hydrophilicity and hydrophobicity, and hence can be strongly adsorbed to the contact lens surface while maintaining an effect of imparting satisfactory hydrophilicity.

### [Other Monomer]

The copolymer (P) may contain a monomer other than the PC monomer and the PME monomer as long as the effect of the present disclosure is not impaired, but is preferably formed only of the PC monomer and the PME monomer.

An example of the other monomer is a polymerizable monomer selected from a linear or branched alkyl (meth)acrylate, a cyclic alkyl (meth)acrylate, an aromatic group-containing (meth)acrylate, a styrene-based monomer, a vinyl ether monomer, a vinyl ester monomer, a hydrophilic hydroxy group-containing (meth)acrylate, an acid group-containing monomer, and a nitrogen-containing group-containing monomer.

Examples of the linear or branched alkyl (meth)acrylate include methyl (meth)acrylate, ethyl (meth)acrylate, propyl (meth)acrylate, butyl (meth)acrylate, lauryl (meth)acrylate, and stearyl (meth)acrylate.

An example of the cyclic alkyl (meth) acrylate is cyclohexyl (meth)acrylate.

Examples of the aromatic group-containing (meth)acrylate include benzyl (meth)acrylate and phenoxyethyl (meth)acrylate.

Examples of the styrene-based monomer include styrene, methylstyrene, and chlorostyrene.

Examples of the vinyl ether monomer include methyl vinyl ether and butyl vinyl ether.

Examples of the vinyl ester monomer include vinyl acetate and vinyl propionate.

Examples of the hydrophilic hydroxy group-containing (meth)acrylate include polyethylene glycol (meth)acrylate, polypropylene glycol (meth)acrylate, 2-hydroxyethyl (meth)acrylate, 2-hydroxybutyl (meth)acrylate, and 4-hydroxybutyl (meth)acrylate.

Examples of the acid group-containing monomer include (meth)acrylic acid, styrenesulfonic acid, and (meth)acryloyloxyphosphonic acid.

An example of the nitrogen-containing group-containing monomer is N-vinylpyrrolidone.

When the copolymer (P) contains the other monomer as a structural unit, a molar ratio among the respective structural units based on the PC monomer (number of moles: nₐ), the PME monomer (number of moles: n_{b}), and the other monomer (number of moles: nₓ) is preferably as follows: nₐ:n_{b}:nₓ=10 to 50:10 to 80:10 to 50.

### [Weight-average Molecular Weight of Copolymer (P)]

The copolymer (P) has a weight-average molecular weight of from 10,000 to 5,000,000, preferably 11,000 or more, more preferably 12,000 or more, still more preferably 13,000 or more, and preferably 4,000,000 or less, more preferably 3,000,000 or less, still more preferably 2,000,000 or less, even more preferably 1,500,000 or less, particularly preferably 1,000,000 or less. The copolymer (P) may have a weight-average molecular weight of, for example, from 10,000 to 2,000,000, from 10,000 to 1,000,000, from 10,000 to 750,000, from 15,000 to 750,000, from 50,000 to 500,000, from 100,000 to 500,000, from 50,000 to 2,000,000, from 100,000 to 2,000,000, from 150,000 to 270,000, or from 13,000 to 1,000,000.

When the weight-average molecular weight is less than 10,000, the ability of the copolymer (P) to be adsorbed to the soft contact lens surface is reduced, and hence there is a risk in that durability of the effect of the copolymer (P) on the soft contact lens surface may not be expected. When the weight-average molecular weight is more than 5,000,000, there is a risk in that an increase in viscosity may cause difficulty in handling.

The weight-average molecular weight of the copolymer (P) refers to a value measured by gel permeation chromatography (GPC) .

### [Production Method for Copolymer (P)]

The copolymer (P) may be prepared by copolymerizing the above-mentioned monomers, and is generally a random copolymer. However, the copolymer (P) may be an alternate copolymer or block copolymer in which the monomers are regularly arranged, and may partially have a graft structure.

Specifically, the copolymer (P) may be obtained by, for example, subjecting a mixture of the above-mentioned monomers to radical polymerization in the presence of a radical polymerization initiator under an inert gas atmosphere, such as nitrogen, carbon dioxide, argon, or helium.

A method for the radical polymerization may be a known method, such as bulk polymerization, suspension polymerization, emulsion polymerization, or solution polymerization. The method for the radical polymerization is preferably solution polymerization from the viewpoint of purification or the like. The purification of the copolymer (P) may be performed by a known purification method, such as a reprecipitation method, a dialysis method, or an ultrafiltration method.

Examples of the radical polymerization initiator may include an azo-based radical polymerization initiator, an organic oxide, and a persulfate.

Examples of the azo-based radical polymerization initiator include 2,2'-azobis(2-diaminopropyl) dihydrochloride, 2,2'-azobis(2-(5-methyl-2-imidazolin-2-yl)propane) dihydrochloride, 4,4'-azobis(4-cyanovaleric acid), 2,2'-azobisisobutyramide dihydrate, 2,2'-azobis(2,4-dimethylvaleronitrile), and 2,2'-azobisisobutyronitrile (AIBN).

Examples of the organic oxide include t-butyl peroxyneodecanoate, benzoyl peroxide, diisopropyl peroxydicarbonate, t-butyl peroxy-2-ethylhexanoate, t-butyl peroxypivalate, t-butyl peroxyisobutyrate, lauroyl peroxide, t-butyl peroxydecanoate, and succinic acid peroxide.

Examples of the persulfate include ammonium persulfate, potassium persulfate, and sodium persulfate.

Those radical polymerization initiators may be used alone or in combination thereof.

The usage amount of the polymerization initiator is generally from 0.001 part by mass to 10 parts by mass, preferably from 0.02 part by mass to 5.0 parts by mass, more preferably from 0.03 part by mass to 3.0 parts by mass with respect to 100 parts by mass in total of the monomers.

The synthesis of the copolymer (P) may be performed in the presence of a solvent. The solvent is not particularly limited as long as the solvent dissolves respective monomer compositions and does not adversely affect the reaction, and examples thereof may include water, an alcohol-based solvent, a ketone-based solvent, an ester-based solvent, a linear or cyclic ether-based solvent, and a nitrogen-containing solvent.

Examples of the alcohol-based solvent include methanol, ethanol, n-propanol, and isopropanol.

Examples of the ketone-based solvent include acetone, methyl ethyl ketone, and diethyl ketone.

An example of the ester-based solvent is ethyl acetate.

Examples of the linear or cyclic ether-based solvent include ethyl cellosolve and tetrahydrofuran.

Examples of the nitrogen-containing solvent include acetonitrile, nitromethane, and N-methylpyrrolidone.

Of those solvents, a mixed solvent of water and an alcohol is preferred.

### [Concentration of Copolymer (P)]

In the treating solution for soft contact lenses of the present disclosure, the concentration of the copolymer (P) is 0.001 w/v% or more, preferably 0.002 w/v% or more, more preferably 0.003 w/v% or more, still more preferably 0.02 w/v% or more, most preferably 0.05 w.v%, and is 2.0 w/v% or less, preferably 1.5 w/v% or less, more preferably 1.0 w/v% or less.

When the concentration of the copolymer (P) is less than 0.001 w/v%, the addition amount of the copolymer (P) is so small that the effect of imparting satisfactory wearing feeling and durability thereof to a contact lens to be obtained is not obtained. When the concentration is more than 2.0 w/v%, there is a risk in that aseptic filtration to be performed in the production of the treating solution for soft contact lenses becomes difficult.

In the present disclosure, "w/v%" is the expression of the mass of a given component in 100 mL of a solution in grams (g). For example, the expression "treating solution of the present disclosure contains 1.0 w/v% of the copolymer (P)" means that 100 mL of the solution contains 1.0 g of the copolymer (P). Water, an alcohol, such as methanol, ethanol, n-propanol, or isopropanol, or a mixed solvent thereof may be used as a solvent to be used for the treating solution of the present disclosure.

Water to be generally used in the production of a pharmaceutical or a medical device may be used as the water to be used for the treating solution for contact lenses of the present disclosure. Specifically, ion-exchanged water, purified water, sterile purified water, distilled water, and water for injection may be used.

### [Other Components]

In the treating solution for contact lenses of the present disclosure, any other component, such as a vitamin, an amino acid, a sugar, a thickening agent, a cooling agent, an inorganic salt, an organic acid salt, an acid, a base, an antioxidant, a stabilizing agent, or an antiseptic agent, may be blended as required in addition to the polymer (P).

Examples of the vitamin include flavin adenine dinucleotide sodium, cyanocobalamin, retinol acetate, retinol palmitate, pyridoxine hydrochloride, panthenol, sodium pantothenate, and calcium pantothenate.

Examples of the amino acid include aspartic acid and salts thereof, and aminoethylsulfonic acid.

Examples of the sugar include glucose, mannitol, sorbitol, xylitol, and trehalose.

Examples of the thickening agent include hydroxypropyl methylcellulose and hydroxyethyl cellulose.

Examples of the cooling agent include menthol and camphor.

Examples of the inorganic salt include sodium chloride, potassium chloride, sodium hydrogen phosphate, and sodium dihydrogen phosphate anhydride.

An example of the organic acid salt is sodium citrate.

Examples of the acid include phosphoric acid, citric acid, sulfuric acid, acetic acid, hydrochloric acid, and boric acid.

Examples of the base include potassium hydroxide, sodium hydroxide, borax, tris-hydroxymethylaminomethane, and monoethanolamine.

Examples of the antioxidant include tocopherol acetate and dibutylhydroxytoluene.

Examples of the stabilizing agent include sodium edetate and glycine.

Examples of the antiseptic agent include benzalkonium chloride, chlorhexidine gluconate, potassium sorbate, and polyhexanide hydrochloride.

### [pH of Treating Solution for Contact Lenses]

The pH of the treating solution for contact lenses of the present disclosure is preferably from 3.0 to 8.0, more preferably from 3.5 to 7.8, still more preferably from 4.0 to 7.6, even more preferably from 4.5 to 7.5, from the viewpoint of improving the wearing feeling.

Herein, the pH of the treating solution for contact lenses refers to a value measured in accordance with the Japanese Pharmacopoeia, Seventeenth Edition, General Tests, Processes and Apparatus, 2.54 pH Determination.

### [Osmotic Pressure and Osmotic Pressure Ratio of Treating Solution for Contact Lenses]

The osmotic pressure of the treating solution for contact lenses of the present disclosure is preferably from 200 mOsm to 400 mOsm, more preferably from 225 mOsm to 375 mOsm, still more preferably from 230 mOsm to 350 mOsm, even more preferably from 240 mOsm to 340 mOsm, from the viewpoint of improving the wearing feeling. The osmotic pressure ratio is preferably from 0.7 to 1.4, more preferably from 0.7 to 1.3, still more preferably from 0.8 to 1.2.

Herein, the osmotic pressure of the treating solution for contact lenses refers to a value measured in accordance with the Japanese Pharmacopoeia, Seventeenth Edition, General Tests, Processes and Apparatus, 2.47 Osmolarity Determination, and the osmotic pressure ratio refers to a value obtained by dividing the obtained value of the osmotic pressure by the value of the osmotic pressure of 0.9 mass% saline (286 mOsm).

### [Production Method for Treating Solution for Soft Contact Lenses]

The treating solution for soft contact lenses of the present disclosure may be produced by a general production method for a solution for contact lenses. The treating solution for soft contact lenses may be produced by, for example, mixing and stirring the polymer (P), and the solvent and the other component as required. The obtained treating solution for soft contact lenses may be subjected to an operation such as aseptic filtration as required.

### [Surface Treatment Method for Soft Contact Lens]

One embodiment of the present disclosure is also directed to a surface treatment method for a soft contact lens including a step of bringing the solution for soft contact lenses of the present disclosure described above into contact with a soft contact lens surface. A method of bringing the solution for soft contact lenses into contact with a soft contact lens surface is not particularly limited, but examples thereof include a method involving immersing a soft contact lens in the solution for soft contact lenses of the present disclosure, and a method involving spraying the solution for soft contact lenses of the present disclosure on a soft contact lens surface.

### [Use of Copolymer (P) for Production of Solution for Soft Contact Lenses]

One embodiment of the present disclosure is also directed to a use of the copolymer (P) described above for production of the solution for soft contact lenses of the present disclosure.

### (Configuration Example of Treating Solution for Soft Contact Lenses of the Present Disclosure)

Examples of combinations of the structural units of the copolymer (P) contained in the treating solution for soft contact lenses of the present disclosure are given below, but the present disclosure is not limited thereto.
Structural unit represented by the formula (1a) :structural unit represented by the formula (1b) :other monomer
2-Methacryloyloxyethyl phosphorylcholine (MPC):structural unit represented by the formula (1b) where "n" represents from 9 to 30
2-Methacryloyloxyethyl phosphorylcholine (MPC):structural unit represented by the formula (1b) where "n" represents from 9 to 30:any one or more other monomers selected from butyl (meth)acrylate, lauryl (meth)acrylate, stearyl (meth)acrylate, 2-hydroxyethyl (meth)acrylate, (meth)acrylic acid, and N-vinylpyrrolidone
2-Methacryloyloxyethyl phosphorylcholine (MPC):structural unit represented by the formula (1b) where "n" represents from 20 to 30
2-Methacryloyloxyethyl phosphorylcholine (MPC):structural unit represented by the formula (1b) where "n" represents from 20 to 30:any one or more other monomers selected from butyl (meth)acrylate, lauryl (meth)acrylate, stearyl (meth)acrylate, 2-hydroxyethyl (meth)acrylate, (meth)acrylic acid, and N-vinylpyrrolidone
In addition, the copolymer (P) contained in the treating solution for soft contact lenses of the present disclosure may be formed by changing each of the structural unit represented by the formula (1a), the structural unit represented by the formula (1b), and the structural unit based on the other monomer in each of the above-mentioned combinations to the structural unit in another combination.

### Examples

The solution for soft contact lenses of the present disclosure is more specifically described below by way of Examples and Comparative Examples. However, the present disclosure is not limited thereto. Copolymers used in Examples and Comparative Examples are as described below.

### <Copolymer (P)>

In Examples, the following copolymers 1 to 4 were used as polymers for Examples.
MPC: 2-methacryloyloxyethyl phosphorylcholine (product of NOF Corporation)
PME-400: methoxy polyethylene glycol methacrylate (n≈9, product of NOF Corporation)
PME-1000: methoxy polyethylene glycol methacrylate (n≈23, product of NOF Corporation)
PME-4000: methoxy polyethylene glycol methacrylate (n≈90, product of NOF Corporation)

Each of the obtained copolymers (1 mg) was dissolved in purified water (1 g), and the resultant solution was subjected to measurement. Other measurement conditions are as described below.
Column: SB-802.5HQ+SB-806M HQ
Mobile phase: 20 mM phosphate buffer solution (pH: 7.0)
Standard substance: polyethylene glycol/oxide
Measurement apparatus: HLC-8320GPC (manufactured by Tosoh Corporation)
Calculation method for weight-average molecular weight: molecular weight calculation program (EcoSEC Data Analysis)
Flow rate: 0.5 mL per minute
Injection volume: 100 µL
Column oven: 45°C
Measurement time: 70 minutes
Copolymer 1: MPC/PME-1000 copolymer (molar ratio=30/70, weight-average molecular weight: 253,000)
Copolymer 2: MPC/PME-1000 copolymer (molar ratio=50/50, weight-average molecular weight: 202,000)
Copolymer 3: MPC/PME-400/PME-1000 copolymer (molar ratio=30/20/50, weight-average molecular weight: 195,000)
Copolymer 4: MPC/PME-4000 copolymer (molar ratio=80/20, weight-average molecular weight: 170,000)

### <Polymer for Comparison>

In Comparative Examples, the following homopolymer 1, homopolymer 2, comparative copolymer, and PVP K90 were used as polymers for comparison.
Homopolymer 1: MPC homopolymer (weight-average molecular weight: 300,000)
Homopolymer 2: PME-1000 homopolymer (weight-average molecular weight: 84,000)
Comparative copolymer: MPC/2-hydroxyethyl methacrylate copolymer (molar ratio=50/50, weight-average molecular weight: 356,000)
PVP K90: polyvinylpyrrolidone K90

### <Test Contact Lens used in Evaluation>

Lens A: 1dayFine UV plus (product of SEED Co., Ltd.) serving as a soft contact lens
Lens B: Menicon 1DAY (product of Menicon Co., Ltd.) serving as a soft contact lens

### (Preparation of Saline)

Saline was prepared referring to a literature (ISO 18369-3:2006, Ophthalmic Optics-Contact Lenses Part 3: Measurement Methods.).

8.3 Grams of sodium chloride, 5.993 g of sodium hydrogen phosphate dodecahydrate, and 0.528 g of sodium dihydrogen phosphate dihydrate were weighed and dissolved in water, and the total amount of the solution was adjusted to 1,000 mL. The solution was filtered to provide saline.

### <Preparation of Treating Solution for Soft Contact Lenses>

The copolymer was dissolved in a predetermined amount of the saline to prepare a polymer solution shown in Table 1 below. Further, the polymer solution and the saline were mixed to prepare a treating solution for contact lenses shown in Table 1 below.

### <Hydrophilicity Evaluation>

In Examples and Comparative Examples, surface hydrophilicity evaluation of a soft contact lens was performed in accordance with the following procedure.
∘Surface Hydrophilicity Evaluation assuming Start of wearing Soft Contact Lens
   (1) 10 mL of the saline was added to a 15 mL conical tube, and one test contact lens taken out of a blister pack was immersed therein, followed by shaking for 6 hours.
   (2) The test contact lens was taken out, and was sealed in a 10 mL glass vial having added thereto 5 mL of the treating solution for soft contact lenses of the present disclosure.
   (3) Sterilization treatment was performed under the conditions of 121°C for 20 minutes.
   (4) The test contact lens was taken out of the glass vial, and the time until a water film on the surface of the lens was broken up (BUT) was measured with a stopwatch. The result was evaluated in accordance with the following criteria.
∘Surface Hydrophilicity Evaluation assuming End of wearing Soft Contact Lens
   (1) 10 mL of the saline was added to a 15 mL conical tube, and one test contact lens taken out of a blister pack was immersed therein, followed by shaking for 6 hours.
   (2) The test contact lens was taken out, and was sealed in a 10 mL glass vial having added thereto 5 mL of the treating solution for soft contact lenses of the present disclosure.
   (3) Sterilization treatment was performed under the conditions of 121°C for 20 minutes.
   (4) 10 mL of the saline was added to a 15 mL conical tube, and the one test contact lens taken out of the glass vial was immersed therein, followed by shaking for 6 hours.
   (5) The test contact lens was taken out of the conical tube, and the time until a water film on the surface of the lens was broken up (BUT) was measured with a stopwatch. The result was evaluated in accordance with the following criteria.
      3 Points: 15 seconds or more
      2 Points: 10 seconds or more and less than 15 seconds
      1 Point: 5 seconds or more and less than 10 seconds
      0 Points: less than 5 seconds

### <Lubricity Evaluation>

In Examples and Comparative Examples, lubricity evaluation of a contact lens was performed in accordance with the following procedure.
∘Lubricity Evaluation assuming Start of wearing Soft Contact Lens
   (1) 10 mL of the saline was added to a 15 mL conical tube, and one test contact lens taken out of a blister pack was immersed therein, followed by shaking for 6 hours.
   (2) The test contact lens was taken out, and was sealed in a 10 mL glass vial having added thereto 5 mL of the treating solution for soft contact lenses of the present disclosure.
   (3) Sterilization treatment was performed under the conditions of 121°C for 20 minutes.
   (4) The test contact lens was taken out of the glass vial, and a friction coefficient of the contact lens in the saline was measured with Nano Tribometer NTR3 (probe material: polypropylene, load: 2 mN, displacement: 1.00 mm, speed: 0.1 mm/s) . The result was evaluated in accordance with the following criteria.
∘Lubricity Evaluation assuming End of wearing Soft Contact Lens
   (1) 10 mL of the saline was added to a 15 mL conical tube, and one test contact lens taken out of a blister pack was immersed therein, followed by shaking for 6 hours.
   (2) The test contact lens was taken out, and was sealed in a 10 mL glass vial having added thereto 5 mL of the treating solution for soft contact lenses of the present disclosure.
   (3) Sterilization treatment was performed under the conditions of 121°C for 20 minutes.
   (4) 10 mL of the saline was added to a 15 mL conical tube, and the one test contact lens taken out of the glass vial was immersed therein, followed by shaking for 6 hours.
   (5) The test contact lens was taken out of the conical tube, and a friction coefficient of the contact lens was measured with Nano Tribometer NTR3 (probe material: polypropylene, load: 2 mN, displacement: 1.00 mm, speed: 0.1 mm/s). The result was evaluated in accordance with the following criteria.
      3 Points: less than 0.50
      2 Points: 0.50 or more and less than 1.0
      1 Point: 1.0 or more and less than 1.5
      0 Points: 1.5 or more

### <Durability Evaluation>

In Examples and Comparative Examples, durability evaluation was performed based on the evaluation results of hydrophilicity and lubricity in accordance with the following criteria.

+++: The scores of the hydrophilicity evaluation and the lubricity evaluation each assuming the start of wearing and the scores of the hydrophilicity evaluation and the lubricity evaluation each assuming the end of wearing were the same.

++: Any one of the scores of the hydrophilicity evaluation and the lubricity evaluation each assuming the start of wearing was decreased by 1 point in the scores of the hydrophilicity evaluation and the lubricity evaluation each assuming the end of wearing.

+: Both of the scores of the hydrophilicity evaluation and the lubricity evaluation each assuming the start of wearing were decreased by 1 point in the scores of the hydrophilicity evaluation and the lubricity evaluation each assuming the end of wearing.

-: Any one of the scores of the hydrophilicity evaluation and the lubricity evaluation each assuming the start of wearing was decreased by 2 points or more in the scores of the hydrophilicity evaluation and the lubricity evaluation each assuming the end of wearing.

### <Wearing feeling Evaluation>

In Examples and Comparative Examples, wearing feeling evaluation was performed based on the evaluation results of hydrophilicity and lubricity assuming the end of wearing in accordance with the following criteria.
Excellent wearing feeling:
   hydrophilicity: 3, lubricity: 3
Satisfactory wearing feeling:
   hydrophilicity: 2, lubricity: 3
   hydrophilicity: 3, lubricity: 2
   hydrophilicity: 2, lubricity: 2
Insufficient wearing feeling:
   other than above

### <Example 1>

100 mL of the saline was weighed, and 1 g of the copolymer 1 was added thereto and dissolved therein by stirring (to provide a polymer solution). 10 mL of the polymer solution was taken out, and the total amount of the solution was adjusted to 100 mL by using the saline, and the solution was subjected to sterilization filtration to prepare a treating solution for soft contact lenses. The test contact lens treated with the treating solution for soft contact lenses was subjected to the evaluations of hydrophilicity, lubricity, and durability. The evaluation results are shown in Table 1.

### <Examples 2 to 5>

The test contact lenses treated with treating solutions for soft contact lenses each prepared in the same manner as in Example 1 except that components whose kinds and amounts are shown in Table 1 were used were subjected to the evaluations of hydrophilicity, lubricity, and durability. The evaluation results are shown in Table 1.

### <Comparative Examples 1 to 4>

The test contact lenses treated with treating solutions for soft contact lenses each prepared in the same manner as in Example 1 except that components whose kinds and amounts are shown in Table 2 were used were subjected to the evaluations of hydrophilicity, lubricity, and durability. The evaluation results are shown in Table 2.

### <Evaluation>

The treating solutions for contact lenses of Examples 1 to 3 were each capable of imparting excellent hydrophilicity and excellent lubricity to a soft contact lens surface in the evaluations assuming the start of wearing and assuming the end of wearing. That is, the treating solutions for contact lenses of Examples 1 to 3 and 5 are each capable of imparting, to a soft contact lens, excellent wearing feeling for a long time period by virtue of excellent hydrophilicity and lubricity, and durability thereof.

The treating solution for contact lenses of Example 4 was capable of imparting satisfactory hydrophilicity and satisfactory lubricity to a soft contact lens surface in the evaluations assuming the start of wearing and assuming the end of wearing. That is, the treating solution for contact lenses of Example 4 is capable of imparting, to a soft contact lens, satisfactory wearing feeling for a long time period by virtue of satisfactory hydrophilicity and lubricity, and durability thereof.

The treating solution for contact lenses of Example 5 was capable of imparting satisfactory hydrophilicity and satisfactory lubricity to a soft contact lens surface in the evaluations assuming the start of wearing and assuming the end of wearing. That is, the treating solution for contact lenses of Example 5 is capable of imparting, to a soft contact lens, satisfactory wearing feeling for a long time period by virtue of satisfactory hydrophilicity and lubricity, and durability thereof.

The treating solution for contact lenses of Example 6 was capable of imparting excellent hydrophilicity and satisfactory lubricity to a soft contact lens surface in the evaluations assuming the start of wearing and assuming the end of wearing. That is, the treating solution for contact lenses of Example 6 is capable of imparting, to a soft contact lens, satisfactory wearing feeling for a long time period by virtue of excellent hydrophilicity and satisfactory lubricity, and durability thereof.

The treating solution for contact lenses of Comparative Example 1 had significantly poor durability, and insufficient wearing feeling.

The treating solution for contact lenses of Comparative Example 2 had insufficient wearing feeling.

The treating solution for contact lenses of Comparative Example 3 had significantly poor durability, and insufficient wearing feeling.

The treating solution for contact lenses of Comparative Example 4 had significantly poor durability, and insufficient wearing feeling.

**Table 1**

| | | Example 1 | Example 2 | Example 3 | Example 4 | Example 5 | Example 6 |
|---|---|---|---|---|---|---|---|
| Copolymer used | | Copolymer 1 | | Copolyme r 2 | Copolyme r 3 | Copolymer 4 | |
| Concentration of polymer solution [%] | | 1.00 | | 1.00 | 3.00 | 1.00 | |
| Blended component [mL] | Polym er solut ion | 10 | 10 | 50 | 50 | 2 | 2 |
| | Salin e | 90 | 90 | 50 | 50 | 98 | 98 |
| Total amount [mL] | | 100 | 100 | 100 | 100 | 100 | 100 |
| Concentration of copolymer in Example [%] | | 0.10 | 0.10 | 0.50 | 1.50 | 0.02 | 0.02 |
| Appearance and properties | | Colorles s and transpar ent | Colorles s and transpar ent | Colorles s and transpar ent | Colorles s and transpar ent | Colorles s and transpar ent | Colorles s and transpar ent |
| Contact lens used | | Lens A | Lens B | Lens B | Lens A | Lens A | Lens B |
| Hydrophili city (BUT [seconds]) | Assum ing start of weari ng | 3 points (21) | 3 points (23) | 3 points (25) | 2 points (14) | 2 points (14) | 3 points (17) |
| | Assum ing end of weari ng | 3 points (18) | 3 points (21) | 3 points (22) | 2 points (11) | 2 points (13) | 3 points (17) |
| Lubricity (Friction coefficien t) | Assum ing start of weari ng | 3 points (0.13) | 3 points (0.12) | 3 points (0.16) | 3 points (0.34) | 2 points (0.74) | 3 points (0.39) |
| | Assum ing end of weari ng | 3 points (0.16) | 3 points (0.14) | 3 points (0.21) | 2 points (0.59) | 2 points (0.91) | 2 points (0.66) |
| Durability | | +++ | +++ | +++ | ++ | +++ | ++ |
| Wearing feeling | | Excellen t | Excellen t | Excellen t | Satisfac tory | Satisfac tory | Satisfac tory |

**Table 2**

| | | Comparative Example 1 | Comparative Example 2 | Comparative Example 3 | Comparative Example 4 |
|---|---|---|---|---|---|
| Copolymer used | | Homopolymer 1 | Homopolymer 2 | Comparative copolymer | PVP K90 |
| Concentration of polymer solution [%] | | 1.00 | 1.00 | 1.00 | 1.00 |
| Blended component [mL] | Polymer solution | 10 | 10 | 10 | 10 |
| | Saline | 90 | 90 | 90 | 90 |
| Total amount [mL] | | 100 | 100 | 100 | 100 |
| Concentration of copolymer in Example [%] | | 0.10 | 0.10 | 0.10 | 0.10 |
| Appearance and properties | | Colorless and transparent | Colorless and transparent | Colorless and transparent | Colorless and transparent |
| Contact lens used | | Lens A | Lens B | Lens B | Lens A |
| Hydrophilicity (BUT [seconds]) | Assuming start of wearing | 3 points (18) | 1 point (9) | 3 points (15) | 3 points (29) |
| | Assuming end of wearing | 0 points (3) | 1 point (7) | 0 points (3) | 0 points (4) |
| Lubricity (Friction coefficient) | Assuming start of wearing | 1 point (1.47) | 1 point (1.08) | 1 point (1.26) | 3 points (0.15) |
| | Assuming end of wearing | 0 points (2.24) | 0 points (1.63) | 0 points (2.10) | 0 points (1.78) |
| Durability | | - | + | - | - |
| Wearing feeling | | Insufficient | Insufficient | Insufficient | Insufficient |

### Industrial Applicability

The use of the treating solution for soft contact lenses of the present disclosure for a soft contact lens can impart excellent wearing feeling all day to the soft contact lens.

## Claims

1. A treating solution for soft contact lenses, comprising 0.001 w/v% to 2.0 w/v% of a copolymer (P),
wherein the copolymer (P) has structural units represented by the following formulae (1a) and (1b), has a molar ratio nₐ:n_{b} between the structural units of 10 to 90:10 to 90, and has a weight-average molecular weight of from 10,000 to 5,000,000: 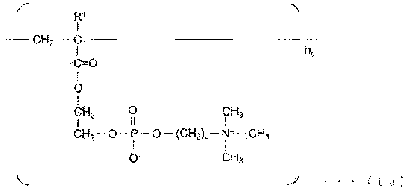 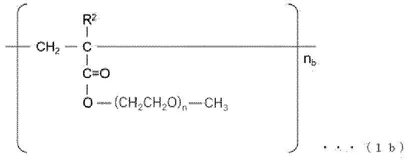 in the formula (1a) and the formula (1b), R¹ and R² each independently represent a hydrogen atom or a methyl group, and in the formula (1b), "n" represents from 4 to 90.

2. The treating solution for soft contact lenses according to claim 1, wherein the structural unit (1a) is 2-(methacryloyloxy)ethyl 2-(trimethylammonio)ethylphosphate.

3. The treating solution for soft contact lenses according to claim 1 or 2, wherein the copolymer (P) is a copolymer formed of the structural units represented by the formulae (1a) and (1b) .
